# EUROPÄISCHE PATENTSCHRIFT

(11) **EP 0 875 198 B1**
(45) Veröffentlichungstag und Bekanntmachung des Hinweises auf die Patenterteilung: **03.07.2002**
(21) Anmeldenummer: 98105277.2
(22) Anmeldetag: 24.03.1998
(51) Int. Cl.: A61B 5/00, A61M 1/34, A61M 1/36

(54) **Einrichtung zur sensorischen Intensivüberwachung eines Patienten**
Means for sensory intensive monitoring of a patient
Dispositif de surveillance intensive sensorielle d'un patient

(30) Priorität: 26.03.1997 DE 29705426 U
(43) Veröffentlichungstag der Anmeldung: 04.11.1998
(73) Patentinhaber: Unger, Roland, Dr., 01827 Graupa (DE); Saxonia Medical GmbH, 01454 Radeberg (DE)
(72) Erfinder: Unger, Roland, Dr., 01827 Graupa (DE)
(74) Vertreter: Ilberg, Roland W., Dipl.-Ing.

(56) Entgegenhaltungen:
- EP-A- 0 087 964
- EP-A- 0 240 101
- DD-A- 136 213
- FR-A- 2 581 316

## Beschreibung

Die Erfindung betrifft eine Einrichtung zur sensorischen Intensivüberwachung von Parametern eines Intensivtherapiepatienten, die in einen im peripheren Kreislauf zeitweilig angelegten extrakorporalen, arteriovenösen Shunt eingefügt ist. Die Intensivüberwachung unterstützend erlaubt die Einrichtung auch eine Intensivbehandlung des Patienten.

Es ist allgemein bekannt und üblich, eine Vielzahl physikalischer Blutparameter von unterschiedlichen Körperregionen über non-invasive Messapparate zu erfassen.

Andere Messverfahren benutzen das invasive Überwachungsprinzip, bei dem in die Gefäßbahn eingeführte Katheter einen oder mehrere Sensoren auf der Katheteroberfläche tragen.

Wichtige biochemische Blutparameter werden durch Blutprobenentnahmen extern in Analyseautomaten bestimmt. Der Nachteil der so angelegten oder eingeführten Sensoren liegt in ihrer zergliederten Platzierung an unterschiedlichen Stellen der Körperoberfläche oder beinhaltet den Zwang zu extremer Miniaturisierung. Die Messung ist damit diskontinuierlich und personell aufwendig.

Eine bekannte Messeinrichtung benutzt den arteriovenösen Überwachungsweg, platziert die Sensoren aber wandständig und formschlüssig integriert am Rande einer laminaren Strömung (DD 123 427, DD 125 917, DD 136 213). Die Nachteile dieser Messapparatur bestehen im zu hohen Shuntvolumen, dem Zwang zur Schaffung kleiner, abgerundeter, konkaver Sensorflächen und in der fehlenden Möglichkeit, biochemische Blutparameter, wie Kreatinin, Harnstoff, Serum-Elektrolyte oder Eiweißkörper, direkt über Sensoren am Blutserum zu messen.

Es ist weiterhin bekannt, während einer Bluttherapierung mittels eines Hämodialysators, vor dem eine Fistel gelegt ist, hämodynamische Parameter zu erfassen, vorzugsweise die Bluttemperatur, um daraus Aufschluß über die ordnungsgemäße Wirkungsweise des Dialysators zu gewinnen, insbesondere über die über eine Blutpumpe realisierte Blutförderrate (DE 195 41 783 C1). Für eine Intensivüberwachung eines Patienten ist die Methode ungeeignet.

Aus der FR-A-25 81 316 ist ein Apparat für eine automatische Kalibrierung der Transmembrandrücke eines Hämofilters zum Zwecke eines optimierten Plasmafiltrationsflusses bekannt, der alle Merkmale des Oberbegriffs des Anspruchs 1 enthält. Mit Hilfe eines Computers, der verschiedene Typen eines Plasmafilters berücksichtigen kann, werden ein schneller Plasmaaustausch erreicht und somit blutdynamische und mechanische Komplikationen vermieden. Mit Hilfe von automatischen Druckmessungen im Zu- und Ablauf eines Plasmafilters und einer entsprechenden Steuerung von Pumpen vor und nach dem Plasmafilter kann die Dauer der Blutbehandlung eines Patienten unter Berücksichtigung eines maximal zulässigen Druckes im Blutkreislauf des Patienten verkürzt werden. Der Apparat wird für eine therapeutische Blutbehandlung eines Patienten eingesetzt und ist ohne die dafür notwendigen Einsatzteile für diagnostische Zielstellungen bezüglich der Kennzeichnung von Blutparametern im Kreislauf eines Intensivtherapiepatienten zum Zwecke einer Intensivüberwachung ungeeignet.

Gegenstand der Erfindung ist eine Einrichtung zur Intensivüberwachung eines Intensivtherapiepatienten, die in einem im peripheren Kreislauf zeitweilig angelegten extrakorporalen, arteriovenösen Shunt eingefügt ist und physikalische, physikalisch-chemische und biochemische Messdaten ohne manuelle Blutentnahmen auf einem hohen Automatisierungsgrad abnimmt. Gleichzeitig sollen in einer Ausgestaltung über diese Einrichtung begleitende therapeutische Handlungen zugelassen sein.

Diese Aufgabe wird erfindungsgemäß durch die in den Ansprüchen angegebene Einrichtung gelärt.

Erfindungsgemäß wird dies dadurch erreicht, dass in einem kurzstreckig, extrakorporal verlaufenden, arteriovenösen Bereich unter Ausnutzung der arteriovenösen Druckdifferenz ein Kapillarfilter für Hämofiltration plaziert wird, wobei in dessen Zu- und Ablaufstrecken Sensoren zur Erfassung physikalischer und physikalisch-chemischer Blutparameter so angeordnet sind, dass sie von der Blutströmung erfasst werden. Das Blut fließt nach Überwindung des Kapillarwiderstandes in das venöse System zurück. Der Kapillarfilter limitiert das Shuntvolumen und erzeugt ein dem Blutserum adäquates Filtrat, das über einen Abfluss in direkter Flussrichtung einem Messkammersystem zur Analyse mittels biochemischer Sensoren zugeführt wird. An den Ausgang der Messkammer ist ein Überlauf für das Abscheiden des Hämofiltrats auch im Rahmen zusätzlicher therapeutischer Handlungen angeordnet.

Die extrakorporal angelegte Messeinrichtung ist modular aufgebaut und vorzugsweise zwischen Arteria radialis und über eine Ellenbogenvene zur Vena jugularis über einen hochgeschobenen Venenkatheter eingeordnet.

Die Dimensionierung des Kapillarfilters ist entsprechend der Aufgabe der Einrichtung im Rahmen der Intensivüberwachung und erforderlichenfalls Intensivtherapie variabel, für reine Überwachungsaufgaben ist der Kapillarfilter klein, für zusätzliche Hämofilteraufgaben größer dimensioniert.

Die Sensoren erfassen vorzugsweise die Messparameter:
- arterieller und venöser Druck
- Shuntflow für Hämodialyse und zur Kreislaufüberwachung
- Temperatur
- EKG-Signal
- Sauerstoffsättigung
- Blutgaswerte (Sauerstoffpartialdruck, Kohlendioxidparti-
- keldruck, ph-Wert)
- Kreatinin oder Harnstoff
- Serum-Elektrolyte
- Eiweißkörper ausgewählter Art

An therapeutischen Handlungen können eine unterstützende Hämofiltration und die Einleitung von Infusionen vorgenommen werden.

Die Vorteile der Einrichtung liegen in einer automatischen, kontinuierlichen und direkten Messung einer Vielzahl von Parametern an nur einer Messstelle ohne manuelle Blutabnahme. Während der Überwachung besteht zugleich die Möglichkeit, ohne weitere Eingriffe in das Blutsystem eine unterstützende Therapie vorzunehmen.

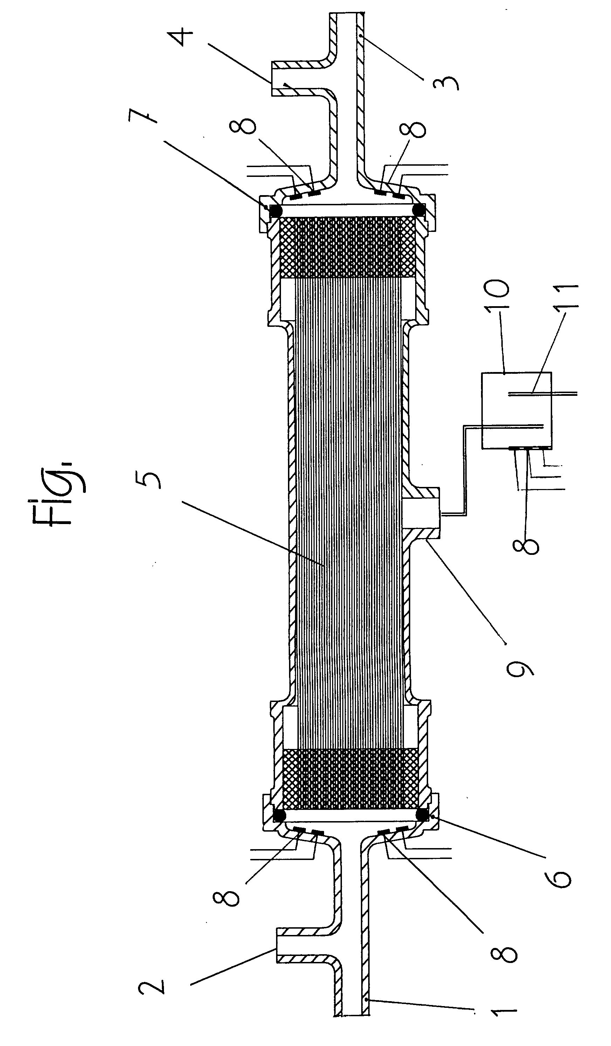

Im folgenden wird die Erfindung anhand eines Beispiels und mit Bezug auf die beiliegende Zeichnung näher erläutert.

Die modular aufgebaute Einrichtung wird vorzugsweise zwischen die bekannten Kanülisierungssysteme der Arterie und der Vene auf der Dorsalseite des Unterarms angeordnet.

Zwischen einem Zulaufrohr 1 mit einem Zulaufstutzen 2 für eine dosierte Zufuhr blutgerinnungshemmender Substanzen und einem Ablaufrohr 3, in das ein Zulaufstutzen 4 für die Einleitung von Infusionen eingebunden ist, ist als zentraler Modul ein spezieller Kapillarfilter 5 mit Hämofilterfunktion angeordnet. Der Kapillarfilter 5 dient zum einen der Begrenzung des Shuntflow durch Widerstandserhöhung und zum anderen der Abtrennung einer Blutserumfraktion zum Zwecke der externen, kontinuierlichen Untersuchung auf biochemische Blutparameter und im Bedarfsfall auch der Ausscheidung von krankhaft angestiegenen Stoffwechselprodukten. Er besteht im wesentlichen aus einem zylindrischen Gehäuse, in das koaxial eine Vielzahl paralleler Hohlfasern eingeführt sind, die mit Ausnahme der offengehaltenen Enden der Hohlfasern endseitig mit Vergussmasse miteinander und mit der Gehäuseinnenwand dicht vergossen werden. Die beiden Gehäuseenden sind mit einer Zulaufkappe 6 und einer Ablaufkappe 7 abgeschlossen, über die der Blutzulauf und Blutablauf erfolgt. In die Zulaufkappe 6 und die Ablaufkappe 7 sind Sensoren 8 zur Messung physikalischer und physikalisch-chemischer Blutparameter radiär so eingebunden, dass sie mit ihren Wirkungsflächen in den turbulenten Strömungsbereich des Kappeninnenraumes hineinragen, ohne den Blutfluss entscheidend zu behindern. Der Kapillarfilter 5 erzeugt ein quantitativ messbares Hämofiltrat. Das Hämofiltrat wird über einen Abflussstutzen 9 einem externen, nur schematisch dargestellten Messkammersystem 10 für biochemische Analysen zugeführt, das Sensoren 8 für die Auswertung biochemischer Messdaten enthält und einen Überlauf 11 für den Abfluss des Hämofiltrats hat.

Die Figur zeigt ein Ausführungsbeispiel in horizontaler, arteriovenöser Flussrichtung. Es ist grundsätzlich aber auch möglich, bei Veränderungen konstruktiver Art an den Zu- und Ablaufelementen die Flussrichtung vertikal zu gestalten. Die einzelnen Sensoren 8 senden drahtgebunden ihre Daten an eine nicht näher dargestellte, externe, rechnergestützte Auswerteeinheit. Ebenso können mehrere Sensoren 8 auf speziellen Computer-Chips integriert werden und ihre Daten drahtlos an die Auswerteeinheit senden.

Für eine reine Überwachungsfunktion kann der Kapillarfilter 5 sehr kleinbauend ausgeführt werden, beispielsweise hat er eine Länge von 10 cm und einen Rohrdurchmesser von 1 cm für wenige Hundert Hohlfasern.

Für eine unterstützende Therapierung sind je nach Art der Therapie größere Kapillarfilter 5 einzusetzen.

### Bezugszeichen

- Zulaufrohr: 1
- Zulaufstutzen: 2
- Ablaufrohr: 3
- Zulaufstutzen: 4
- Kapillarfilter: 5
- Zulaufkappe: 6
- Ablaufkappe: 7
- Sensor: 8
- Abflussstutzen: 9
- Messkammersystem: 10
- Überlauf: 11

## Patentansprüche

1. Einrichtung zur sensorischen Intensivüberwachung von Parametern eines Intensivtherapiepatienten in einem im peripheren Kreislauf zeitweilig angelegten extrakorporalen, arteriovenösen Shunt, wobei die Einrichtung einen Kapillarfilter (5) für Hämofiltration und im Wege der Zuund/oder Ablaufstrecken dieses Kapillarfilters (5) geeignet angeordnete Sensoren (8) zur Messwerterfassung physikalischer, physikalisch-chemischer und biochemischer Blutparameter des Intensivtherapiepatienten enthält, wobei der Kapillarfilter mit einer Zulaufkappe und einer Ablaufkappe ausgebildet ist und **dadurch gekennzeichnet, dass** die biochemischen Parameter in einer Messkammer (10) erfasst werden, die an einen Ablaufstutzen (9) des Kapillarfilters (5) für das Hämofiltrat angeschlossen ist.

2. Einrichtung nach Anspruch 1, **dadurch gekennzeichnet, dass** an den Ausgang der Messkammer (10) ein Überlauf (11) für das Abscheiden des Hämofiltrats auch im Rahmen therapeutischer Handlungen angeordnet ist.

3. Einrichtung nach Anspruch 1, **dadurch gekennzeichnet, dass** der Kapillarfilter zylinderförmig ist und dass Sensoren (8) radiär um eine Ein- und eine Ausströmöffnung inden Kappen (6, 7) des zylinderförmigen Kapillarfilters (5) angeordnet sind.

4. Einrichtung nach Anspruch 1, **dadurch gekennzeichnet, daß** einströmseitig ein Zulaufstutzen (2) für blutgerinnungshemmende Substanzen und/oder ausströmseitig ein Zulaufstutzen (4) für Infusionslösungen mündet.

5. Einrichtung nach Anspruch 1, **dadurch gekennzeichnet, daß** die Sensoren (8) an eine rechnergestützte Auswerteeinheit angeschlossen sind.

6. Einrichtung nach Anspruch 5, **dadurch gekennzeichnet, daß** Sensoren (8) auf einem Mikrochip integriert sind und ihre Daten drahtlos an eine Auswerteeinheit senden.

## Claims

1. Device for sensory intensive monitoring of parameters of an intensive care patient in an extracorporeal, arteriovenous shunt temporarily placed in the peripheral circulation, the device containing a capillary filter (5) for hemofiltration and sensors (8) suitably arranged in the path of the feed and/or discharge sections of this capillary filter (5) to detect readings of physical, physico-chemical and biochemical blood parameters of the intensive care patient, the capillary filter being designed with a feed cap and a discharge cap and **characterised in that** the biochemical parameters are detected in a measuring chamber (10) which is connected to a discharge nozzle (9) of the capillary filter (5) for the hemofiltrate.

2. Device according to claim 1, **characterised in that** an overflow (11) for the separation of the hemofiltrate even in the course of therapeutic treatments is arranged at the outlet of the measuring chamber (10).

3. Device according to claim 1, **characterised in that** the capillary filter is cylindrical and **in that** sensors (8) are arranged in a radial manner around an inlet and outlet aperture in the caps (6, 7) of a cylindrical capillary filter (5).

4. Device according to claim 1, **characterised in that** a feed nozzle (2) for blood clot-inhibiting substances opens on the inlet side and/or a feed nozzle (4) for infusion solutions opens on the outlet side.

5. Device according to claim 1, **characterised in that** the sensors (8) are connected to a computer-assisted evaluation unit.

6. Device according to claim 5, **characterised in that** sensors (8) are integrated on a microchip and transmit their data in a wireless manner to an evaluation unit.

## Revendications

1. Dispositif pour la surveillance intensive sensorielle des paramètres d'un patient soumis à des soins intensifs, inséré dans une dérivation artérioveineuse extracorporelle placée temporairement dans la circulation périphérique, le dispositif contenant un filtre capillaire (5) destiné à l'hémofiltration et, disposés sur les trajets d'amenée et/ou de sortie de ce filtre capillaire (5), des capteurs (8) adaptés à l'enregistrement de paramètres sanguins physiques, physico-chimiques et biochimiques du patient soumis à des soins intensifs, le filtre capillaire comprenant une calotte d'amenée et une calotte de sortie,
**caractérisé en ce que**
les paramètres biochimiques sont enregistrés dans une chambre de mesure (10) reliée à un raccord de sortie (9) du filtre capillaire (5) pour le filtrat sanguin.

2. Dispositif selon la revendication 1,
**caractérisé en ce que**
à la sortie de la chambre de mesure (10), est placé un déversoir (11) destiné à la séparation du filtrat sanguin dans le cadre de l'administration de soins.

3. Dispositif selon la revendication 1,
**caractérisé en ce qu'**
le filtre capillaire a une forme cylindrique, les capteurs (6) sont placés radialement autour d'une ouverture d'entrée et de sortie dans les calottes (6, 7) du filtre capillaire (5) de forme cylindrique.

4. Dispositif selon la revendication 1,
**caractérisé en ce que**
un raccord d'entrée (2) débouche du côté de l'entrée pour des substances anticoagulantes, et/ou un raccord d'entrée (4) destiné aux solutions de perfusion du côté de la sortie.

5. Dispositif selon la revendication 1,
**caractérisé en ce que**
les capteurs (8) sont reliés à un appareil exploitation commandé par ordinateur.

6. Dispositif selon la revendication 5,
**caractérisé en ce que**
des capteurs (8) sont intégrés sur une puce informatique et envoient sans câble leurs données à l'appareil d'exploitation.
